# EUROPÄISCHE PATENTSCHRIFT

(11) **EP 0 786 525 B1**
(45) Veröffentlichungstag und Bekanntmachung des Hinweises auf die Patenterteilung: **04.06.2003**
(21) Anmeldenummer: 96114529.9
(22) Anmeldetag: 11.09.1996
(51) Int. Cl.: C12Q 1/04

(54) **Verfahren zur Vebesserung des Wachstums und des kolorimetrischen Nachweises von Bakterien, Hefen, Pilzen oder Kokken**
Method for improving the growth and the colorimetric determination of bacteria, yeast, fungi or coccus
Méthode pour améliorer la croissance et la détermination colorimétrique des bactéries, des leuvres, des champignons ou des coccus

(30) Priorität: 24.01.1996 DE 19602345
(43) Veröffentlichungstag der Anmeldung: 30.07.1997
(73) Patentinhaber: BIOTEST AG, D-63303 Dreieich (DE)
(72) Erfinder: Horn, Jürgen, Dr., 63329 Egelsbach (DE)
(74) Vertreter: Strych, Werner Dr.

(56) Entgegenhaltungen:
- EP-A- 0 167 724
- EP-A- 0 223 685
- DE-C- 4 316 394
- US-A- 3 983 003
- US-A- 4 168 204

## Beschreibung

Die vorliegende Erfindung betrifft ein Verfahren zur Verbesserung des Wachstums und des Nachweises von Bakterien, Hefen, Pilzen oder Kokken, indem man dem Kulturmedium sterilfiltrierten Hefeextrakt und p- lodonitrotetrazoliumviolett hinzusetzt

Das Verfahren ist insbesondere zum Nachweis von Mykobakterien oder Keimen unter Streßbedingungen, wie Luftkeimen nach dem Streß des Austrocknens in der Luft, geeignet.

Besondere Ausgestaltungen des erfindungsgemäßen Verfahrens sind in den Unteransprüchen beschrieben.

Das Wachstum von Bakterien, Hefen, Pilzen, Kokken bzw. Keimen erfolgt üblicherweise auf dafür bekannten Kulturmedien. Ihr Nachweis kann dann z.B. mittels geeigneter Indikatoren auf kolorimetrischem Weg vorgenommen werden.

Für Bakterien, Kokken und andere Keime verwendet man allgemeine Nährmedien, wie z.B. Tryptic SOY Agar oder Bouillon, Hefen und Pilze können z.B. auf Sabourand Agar, Bouillon oder RMPI 1640 Bouillon gezüchtet werden. Übliche Methoden für Mykobakterien sind solche auf Ei- oder Eigelbbasis wie

Löwenstein Jensen oder Stonebrink oder Agar Medien wie Middlebrook 7H10, 7H11 oder flüssige Medien wie Middlebrook 7H oder Kirchner Medium mit 10% Pferdeserum (DIN 58 943-3, Manual of Clinical Microbiology, 6th ed., 414-416).

Als Wachstumsnachweis kann hierzu die Verwertung von C14 palmitinsäure dienen, die radioaktives 14 CO2 freisetzt (Bactec, Manual of Clinical microbiology 6th ed. 415), ferner der Sauerstoffverbrauch im Medium, der durch einen Fluoreszenzindikator angezeigt wird (EP-A 0 509 781 Al, Handelsnahme des Produktes MGIT) oder durch barometrische Messung (ESP Automat für Blutkultur und Mykobakterien, Difco) bzw. durch Redoxindikatoren wie Resazurin/Methylenblau (DE 4 316 394) oder Tetrazoliumchlorid (Dtsch. Med. Wochenschrift 75,1471 (1995) erfolgen.

Das Wachstum von Bakterien erfolgt relativ langsam. So benötigen Mykobakterien auf festen Medien je nach Menge der aufgeimpften Mykobakterien etwa 2-6 Wochen, in flüssigen Medien entsprechend 1-3 Wochen. Der Wachstumsnachweis mit zusätzlichen Geräten ist sehr kostenaufwendig, ebenso die Beseitigung des radioaktiven Abfalls. Bei bekannten kolorimetrischen Redoxindikatoren wie Resazurin/Methylenblau oder Tetrazoliumchlorid zeigt sich der Effekt, daß z.B. klinische Stämme von Mykobakterien tuberculosis bei niedrigen Keimzahlen gehemmt werden und erst bei größeren Keimmengen zufriedenstellend wachsen. Es müssen jedoch auch niedrige Keimzahlen im klinischen Probematrial zuverlässig nachgewiesen werden. Darüberhinaus sind diese Indikatoren in hohen Mengen, welche für geringe Keimzahlen erforderlich sind, toxisch. Eine Mengenreduzierung dieser kolorimetrischen Redoxindikatoren verringert zwar die Toxizität, führt jedoch dazu, daß klinische Isolate z.B. von Mykobakterien tuberculosis zwar noch wachsen, jedoch nicht mehr gefärbt sind, d.h. der gewünschte kolorimetrische Nachweis ausbleibt.

Bei der Isolierung und dem Nachweis von Luftkeimen unterschiedlicher Art, wie z.B. Bakterien, Kokken, Hefen, Pilze und Sporen, treten folgende Probleme auf: Luftkeime sind durch die Austrocknung in der Luft gestreßt Darüberhinaus werden die Medien zum Nachweis der Keime häufig gammasterilisiert, was wiederum Streß für das Medium bedeutet und daher zum schlechteren Wachstum der nachzuweisenden Keime führt. Beide Faktoren führen dazu, daß Keime bestimmter Arten, insbesondere von grampositiven Bakterien, und Kokken, nicht mehr zuverlässig auf üblichem Weg (kolorimetrisch mit Resazurin) nachgewiesen werden können.

Die US-A 3 983 003 beschreibt ein Medium zum Nachweis von Mykobakterien, welches Hefeextrakt und insbesondere Hyaluronsäure enthält. Die teure Hyaluronsäure wird zugesetzt, um das Bakterienwachstum zu verbessern.
In EP-A 0 223 685 werden Medien zur Durchführung der Decarboxylase-Tests bei Nonfermentern beschrieben.
EP-A 0 167 724 betrifft ein Medium zum Nachweis einer Malonatverstoffwechselung von zu prüfenden Mikroorganismen, weiches u.a. Hefeextrakt aufweist.
Hiermit ist wie mit obengenanntem Decarboxylase-Test lediglich der positiv/negativ-Nachweis als selektive Differenzierung möglich, nicht jedoch ein allgemein empfindlicher Nachweis von niedrigen Keimzahlen.
DE-C-43 16 394 beschreibt ein Verfahren zum optischen Nachweis des Wachstums von Mikroorganismen durch visuelle Ermittlung des Farbumschlags eines Systems aus Methylenblau und Resazurin.
In "Mikrobieller Diagnostik", Ed. Friedrich Burkhardt, Thieme Verlag, 1992, wird ein Medium zur Züchtung von Mykoplasmen beschrieben, das neben anderen Komponenten, Hefeextrakt beinhaltet. Dieser Hefeextrakt wird hergestellt durch 15 minütiges Aufkochen in Wasser und nachfolgendes Autoklavieren bei 121°C oder sterilfiltrieren.

Aufgabe vorliegender Erfindung ist es daher, ein Verfahren zu entwickeln, durch das das Wachstum von Bakterien, Pilzen, Hefen, Kokken verbessert und der Nachweis der zu untersuchenden Spezies zuverlässig und kostengünstig erfolgen kann.

Diese Aufgabe wird erfindungsgemäß dadurch gelöst, daß man dem Kulturmedium sterilfiltriertem Hefeextrakt und p-lodonitrotetrazoliumviolett hinzufügt.

Überraschenderweise zeigte sich, daß sich durch den erfindungsgemäßen Zusatz von sterilfiltriertem Hefeextrakt und p- lodonitrotetrazoliumviolett das Wachstum von Bakterien, Pilzen, Hefen, Kokken beschleunigen läßt Wird der Redoxindikator p-lodonitrotetrazoliumviolett (INT) hinzugesetzt, kann das Wachstum der zu untersuchenden Spezies auf einfache Weise kolorimetrisch nachgewiesen werden.

Insbesondere geeignet ist das erfindungsgemäße Verfahren zum Nachweis von Mykobakterien und insbesondere klinischer Mykobakterien tuberculosis Isolate, die auf verschiedenen Medien wie 7H9 Bouillon, 7H 1 2, 7H9 mit OADC «Oleic Acid, Albumin, Dextrose, Catalase, Trademark, siehe Difco Manual) und PANTA (Polymyxin, Amphatericin B, Nalidixic Acid, Trimethroprim, Azlccillin) im MGIT®-System Mycobacteria Growth Indicator Tube); in Kirchner-Medium mit Pferdeserum sowie auf festen Medien wie Löwenstein Jensen wachsen.

Selbst durch lange Aufbewahrungszeiten geschädigte Bakterien, insbesondere Mykobakterien, wachsen mit dem erfindungsgemäßen Zusatz besser heran.

Die hierfür benötigte Zeit kann hierbei erheblich reduziert bzw. der Nachweis selbst vereinfacht werden.

So kann z.B. die Empfindlichkeitstestung von Mykobakterien, die sonst nur unter Verwendung eines radioaktiven Substrates (14_{c}-Palmitinsäure im System Bactec ® 460 von Becton Dickinson) innerhalb 1 Woche möglich ist, durch Zusatz von sterilfiltriertem Hefeextrakt zu 7H9 Bouillon bereits visuell als Trübungsmessung innerhalb von 5 bis 7 Tagen ausgewertet werden, ohne daß kostenträchtige Systeme, wie der Radioaktivitätsnachweis, mit den damit verbundenen Entsorgungsproblemen erforderlich wären.

Der erfindungsgemäße Zusatz eignet sich auch besonders zum Nachweis von Luftkeimen, wobei die erfolgte Keimzahlerhöhung durch sterilfiltrierten Hefeextrakt zu einem schnelleren Wachstum allgemein und insbesondere zu einem schnelleren Wachstum als bei nichtgammasterilisierten Medien führt. Somit wird auch der kolorimetrische Nachweis der Keime, insbesondere von gramnegativen Bakterien, Hefen und Pilzen, möglich. Dies ermöglicht auch die gamma-Sterilisierung von Medien wobei der sterilfiltrierte Hefeextrakt die Schädigung der Medien in Bezug auf die Wachstumseigenschaften kompensiert. Damit wachsen Keime auf gammasterilisierten Medien mit sterilfiltriertem Hefeextrakt sogar noch leicht besser als auf herkömmlichen, nicht gamma-sterilisierten Medien.

Als Grundmedium kann beispielsweise Tryptic Soy Agar oder ähnliche Nährmedien eingesetzt werden.

Der erfindungsgemäße Zusatz von sterilfiltriertem Hefeextrakt erfolgt bevorzugt in flüssiger Form in Mengen von 0,5 bis 10/1, insbesondere 0,5-5 g/1 und ganz besonders bevorzugt 2-2,5 g/l.

Der Indikator lodonitrotetrazoliumviolett (INT) wird dem Kulturmedium bevorzugt in einer Menge von 1 bis 30 mg/l, insbesondere 5-20 mg/1 und insbesondere 8-15 mg/1 hinzugefügt.

Das Wachstum läßt sich auch in anderen kommerziellen Systemen durch den nachträglichen Zusatz der erfindungsgemäßen Kombination beschleunigen,wie im nachfolgendem Beispiel 4 mit MGIT® mit sterilfiltriertem Hefeextrakt + INT gegen Beispiel 3 (kommerzielles MGIT® System) gezeigt

Durch den erfindungsgemäßen Zusatz kann somit insgesamt das Wachstum von Bakterien, Hefen, Kokken, Pilzen beschleunigt und darüberhinaus auf aufwendige Nachweisapparaturen verzichtet werden.

Die Erfindung wird anhand der nachfolgenden Beispiele näher erläutert.

### I. Beispiele 1- 4

Auf verschiedenen bekannten Medien (fest oder flüssig) wurde das Wachstum von Mykobakterien tuberculosis in verschiedenen Beiimpfungsmengen getestet Hierbei enthalten die Medien 2 und 4 den erfindungsgemäßen Zusatz an sterilfiltriertem Hefeextraktakt und INT.

In Tabelle I sind die Ergebnisse dieses Versuchs wiedergegeben. Wie hieraus ersichtlich ist, wird mit dem erfindungsgemäßen Zusatz gemäß der Probe 2 ein schnelleres Wachstum gegenüber 1 (ohne Zusatz) und bei 4 (Erfindung) gegenüber 3 (Vergleich) erzielt.

### Medien zur Isolierung von Mykobakterien (Beispiele 1-4)

| | 1. Kirchner Medium normal | 2. Kirchner Medium mit Hefeextrakt und INT |
|---|---|---|
| dinatriumhydrogenphospat-12-hydrat | 3,0 g | 3,0 g |
| Kaliumdihydrogenphosphat | 4,0 g | 4,0 g |
| Magesiumsulfat | 0,6 g | 0,6 g |
| Natriumcitrat | 2,5 g | 2,5 g |
| L-Asparagin | 0,1 g | 0,1g |
| Kaliumaspartat | 4,0 g | 4,0 g |
| D-Alanin | 2,0 g | 2,0 g |
| Na-pyruvat | 0,5 g | 0,5 g |
| Hemin (in 1 ml NaOH gelöst) | 1,66 mg | 1,66 mg |
| Ammoniumeisen (III) sulfat | 30 mg | 50 mg |
| Aqua dest | 900 ml | 800 ml |
| Glycerin separat autoklaviert | 20 ml | 20 ml |
| Pferdeserum (1 bei 56°C inaktiviert) | 100 ml | 100 ml |
| Katalaselösung (Boehringer Mannheim 106836) | 5 ml | 5 ml |
| Ribonucleinsäurelösung 2,5 ml mit 25 µg | 25 µg | 25 µg |
| Hefeextrakt serilfiltriert | - | 2,5 g in 100 ml H₂O |
| INT Solution (Sigma 9405) INT = p-Iodonitrotetrazoliumviolet = (2-[-Iodophonyl]-3-(4-nitrophenyl]-5 phenyltetrazoliumchlorid | - | 24 ml |

Die ersten 12 Komponenten werden zusammen autoklaviert, die weiteren als sterile Lösungen sterilfiltriert (ohne Autoklavieren) zugesetzt.
3. MGIT® von Becton Dickinson
   4 ml 7H9 Bouillon
   +OADC
   + PANTA
   alle Komponenten von Becton
   Dickinson
4. MGIT® mit sterilfiltiertem
   Hefeextrakt und INT
   4 ml 7H9 Ecuillon
   + OADC
   + PANTA
   + 10 mg Hefeextrakt sterilfiltriert in 0,4 ml H₂O

**Tabelle I**

| Wachstum von Mykobakterium tuberculosis in verschiedenen Beimpfungsmengen auf den angegebenen Medien | | | | | | |
|---|---|---|---|---|---|---|
| Stamm Inokulum meng | | | | Wachstum auf Medium Nr in Tagen | | |
| | 1 | | 2 | 3 | | 4 |
| M. tuberculosis H37RV Mcfarland 0.5 x | | | | | | |
| 10⁻² | 7 | | 5 lila | 6 | | 5 lila |
| 10⁻³ | 11 | | 8 lila | 9 | | 7 lila |
| 10⁻⁴ | 14 | | 11 d.lila | 12 | | 10 d.lila |
| 10⁻⁵ | 16 | | 12 d.lila | 14 | | 12 d.lila |
| 10⁻⁶ | 23 | | 15 d.lila | 17 | | 14 d. lila |
| 10⁻⁷ | 28 | | 19 lila | 20 | | 18 d.lila |

| M. tuberculosis klin. 1 Mcfariand 0.5 x | | | | | | |
|---|---|---|---|---|---|---|
| 10⁻² | 9 | | 6 lila | 8 | | 6 lila |
| 10⁻³ | 13 | | 8 lila | 12 | | 9 lila |
| 10⁻⁴ | 17 | | 10 d.lila | 16 | | 11 d.lila |
| 10⁻⁵ | 22 | | 13d.lila | 21 | | 14 d.lila |
| 10⁻⁶ | 25 | | 16 d.lila | 25 | | 17 d.lila |
| 10⁻⁷ | 32 | | 20 d.lila | 30 | | 20 d.lila |

| M. tuberculosis klin. 15 Mcfarland 0.5 x | | | | | | |
|---|---|---|---|---|---|---|
| 10⁻² | 8 | | 5 lila | 6 | | 5 lila |
| 10⁻³ | 12 | | 8 lila | 9 | | 8 lila |
| 10⁻⁴ | 15 | | 11 d.lila | 12 | | 11 d.lila |
| 10⁻⁵ | 22 | | 13 d.lila | 14 | | 12 d.lila |
| 10⁻⁶ | 26 | | 16 d.lila | 18 | | 15 d.lila |
| 10⁻⁷ | 29 | | 19 d.lila | 22 | | 18 d.lila |
| * bei 3 Auswertung mit Fluoreszenz nach Vorschrift zu MGIT® bei 4 Auswertung mit Fluoreszenz nach Vorschrift zu MGIT® und zusätzlich Auftreten lila gefäfbter Kolonien (durch INT). d.lila= dunkellila 2, = Erfindung 1*, 3* = Vergleich | | | | | | |

### II. Beispiele 5 - 6

In diesen Beispielen wurde die Empfindlichkeitstestung zum Nachweis von Mykobakterien analog zu dem in DIN 58943, Teil 8 beschriebenen Verfahren für feste Medien mit den flüssigen Medien 5 , und 6 bis 6 C angesetzt. Die Auswertung erfolgt durch Feststellung von Trübung in der Wachstumskontrolle (sowie bei Resistenz auch durch Trübung) bei den Medien 5.

Bei den Medien 6 bis 6C wird durch Auftreten von lila gefärbten Kolonien Resistenz bzw. Wachstum ausgewertet.

Verwendet wird 7H9 Medium mit ADC enrichment (Standard) und erfindungsgemäß 7H9 Medium mit ADC enrichment + sterilfiltriertem Hefeextrakt + INT.

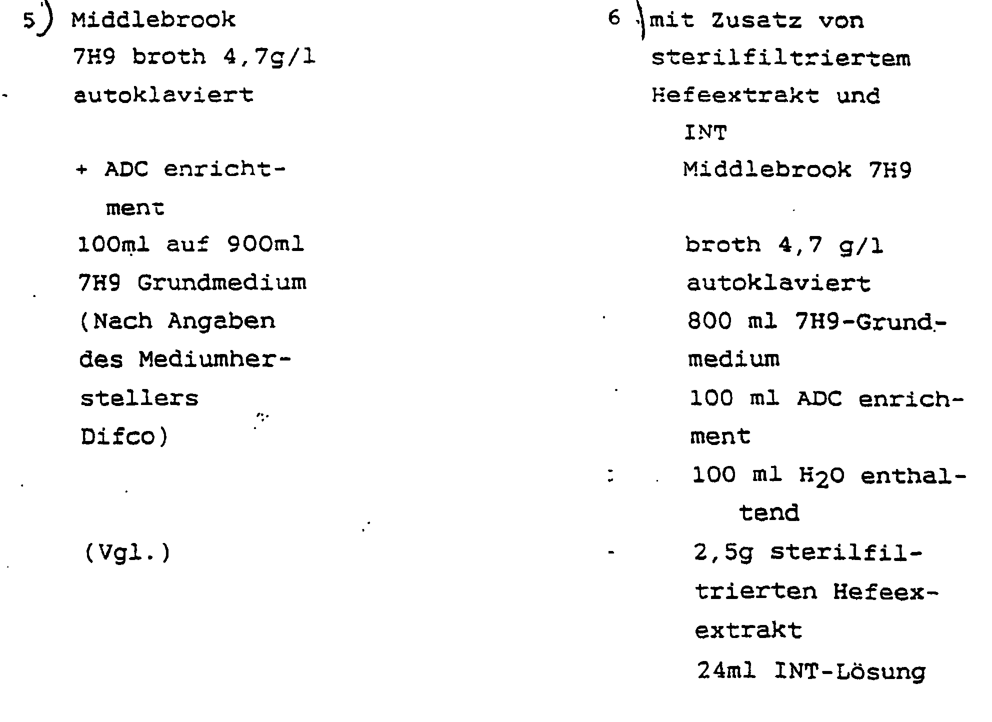

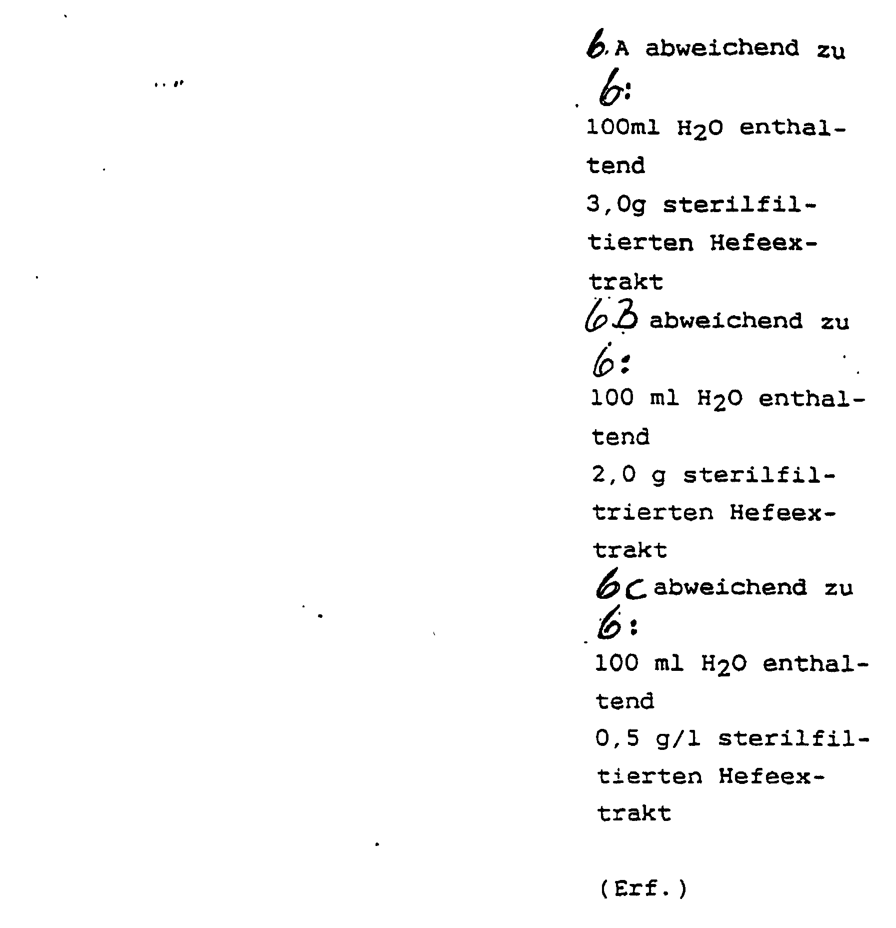

Die Ergebnisse sind in nachfolgender Tabelle II wiedergegeben.

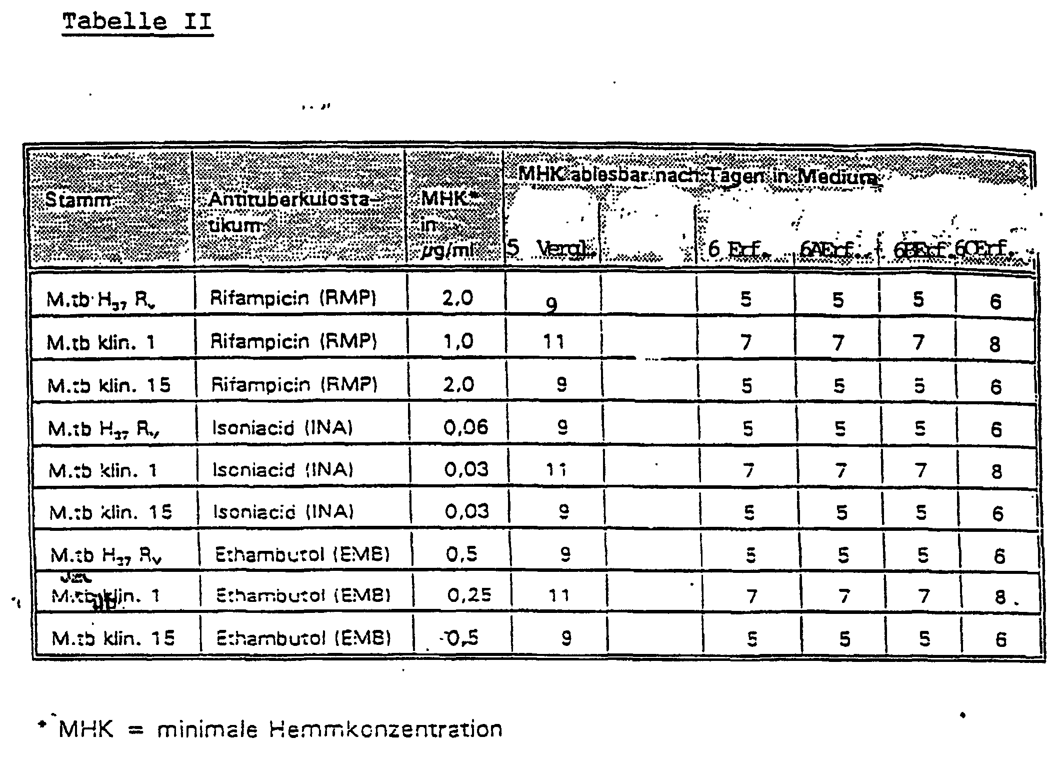

Wie hieraus ersichtlich ist, kann die Probe 6 mit dem erfindungsgemäßen Zusatz schneller ausgewertet werden als im Vergleichsmedium 5.

Die Beispiele 6A - 6C zeigen unterschiedliche Mengen an zugesetztem Hefeextrakt.

### III. Beispiele 7 - 8

In den nachfolgenden Beispielen 7-8 sind Medien mit (Beispiel 8 ) und ohne erfindungsgemäßen Zusatz (7) aufgeführt, welche mit (7A) und ohne (7, 8) vorherige Gammasterilisation (Gammastrahlen von 20-30 Kilogray) als Wachstumsmedium für verschiedene, in Tabelle III genannte Luftkeime eingesetzt wurden.
7.) Tryptic Soy Agar
   zur Keimzahlbestimmung
   15 g Pancreatic Digest of Casein
   5 g Soy Bean Pepton
   5 g NaCl
   0,7 g Lecithin
   7 g Polysorbat 80
   17 g Agar
   1000 ml H₂O
   dasGemisch wird autoviert
8.) Trypic Soy Agar
   zur Keimzahlbestimmung mit
   sterilfiltdirertem Hefeextrakt und INT
   15 g Pancreatic Digest of Casein
   5 g Soy Bean Pepton
   5 g NaCl
   0,7 g Lecithin
   7 g Polysorbat 80
   17 g Agar
   900 ml H₂O
   das Gemisch wird autoklaviert
   100 ml H₂O, enthaltend 2,5 g sterilfiltrierten Hefeextrakt steril zusetzen
   24 ml INT-Lösung steril zusetzen.

Wie aus Tabelle III ersichtlich ist, zeigt Medium 8 (Erf.) eine wesentliche Verbesserung in der Anzahl der wiedergefundenen Kolonien gegenüber 7 (Vgl.).

Ferner kann man sehen daß durch den Streß der gamma-Bestrahlung (Medien 7A) die Anzahl der wiedergefundenen Kolonien gegenüber den nichtbestrahlten Medien ( 7) abnimmt.

**Tabelle III**

| Stamm Inokulummenge | Wachstum auf medium Nr., Anzahl Kolonien nach 1 Tage | | |
|---|---|---|---|
| | 7 | 7A | 8 |
| Bacillus subtilis | | | |
| 10² | 80 | 11 | 58 lila Kolonien |
| 2x10² | 10 | 1 | 0 lila Kolonien |
| 50 | 1 | 0 | 0 lila Kolonien |

| Staph, aureus ATCC 6538 | | | |
|---|---|---|---|
| 10³ | Rasen | 68 | 116 lila Kolonien |
| 2x10² | 138 | 27 | 2 lila Kolonien |
| 50 | 32 | 1 | 0 lila Kolonien |

| Candida pseudotropicalis | | | |
|---|---|---|---|
| 10³ | 17 | 2 | 48 lila Kolonien |
| 2x10² | 3 | 0 | 10 lila Kolonien |
| 50 | 1 | 0 | 3 lila Kolonien |

| Alteromonas putrefaciens | | | |
|---|---|---|---|
| 10³ | Rasen | 59 | Rasen, lila |
| 2x10² | 66 | 21 | 128 lila Kolonien |
| 50 | 11 | 2 | 48 lila Kolonien |

### IV. Klinische Versuche

Mit verschiedenem 'Nährmedium und einem Medium, enthaltend den erfindungsgemäßen Zusatz von sterilfilriertem Hefeextrakt und INT, wurden klinsiche Versuche mit verschiedenen Probenmaterialien zum Nachweis von Mykobacterium tuberculosum durchgeführt.

Die Ergebnisse sind in nachfolgender Tabelle IV wiedergegeben.

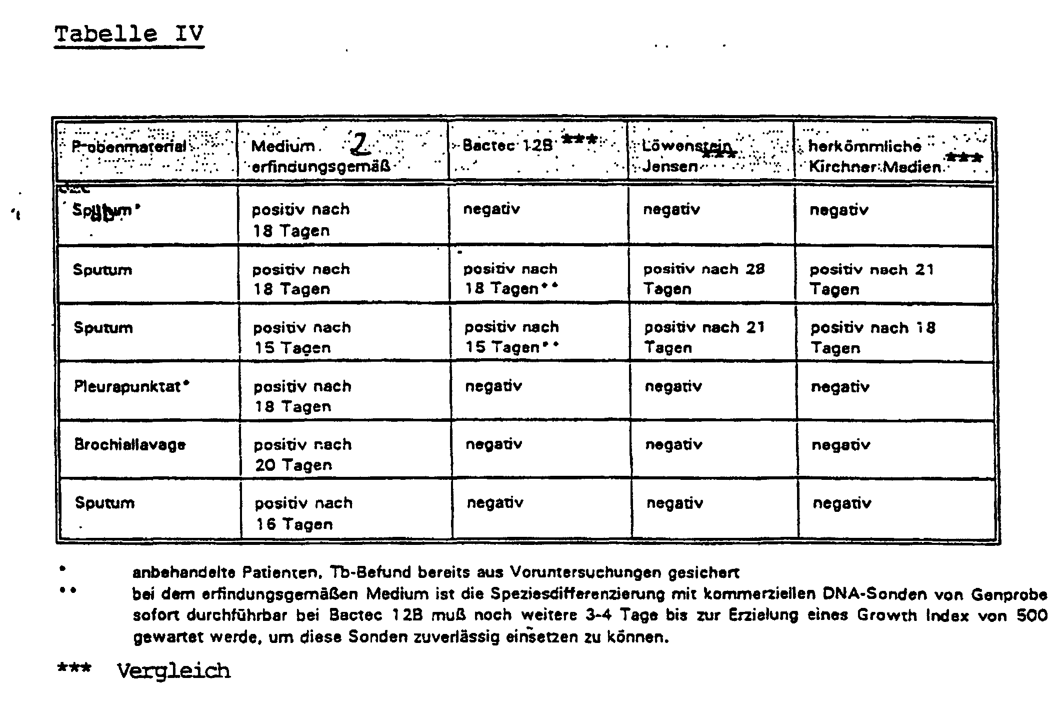

Tabelle IV zeigt, daß durch Anbehandlung geschädigte und gestreßte Keime in dem erfindungsgemäßen Medium noch anzüchtbar sind, während dies in den Vergleichsmedien nicht mehr der Fall ist.

Weiter ist die sofortige Weiterverarbeitung mit molekularbiologischen DNA-Sonden mit dem erfindungsgmäßen Medium am selben Tag, an dem der positive Befund des Wachstums erhoben wird, möglich. Damit ist die Speziesdifferenzierung und der sofortige Befund an den Kliniken möglich, während in traditionellen Systemen noch weiter gewartet werden muß, um genügend Keime zur Untersuchung mit kommerziellen DNA-Sonden zu haben.

Wie aus diesen Beispielen klar hervorgeht, führt das erfindungsgemäße Verfahren in einfacher, kostengünstiger Weise zu zuverlässigen schnellen Ergebnissen bezüglich des Wachstums und dessen Nachweises verschiedenster Spezies.

### IV. Klinische Versuche

Mit verschiedenem Nährmedium und einem Medium, enthaltend den erfindungsgemäßen Zusatz von sterilfilriertem Hefeextrakt und INT, wurden klinsiche Versuche mit verschiedenen Probenmaterialien zum Nachweis von Mykobacterium tuberculosum durchgeführt.

Die Ergebnisse sind in nachfolgender Tabelle IV wiedergegeben.

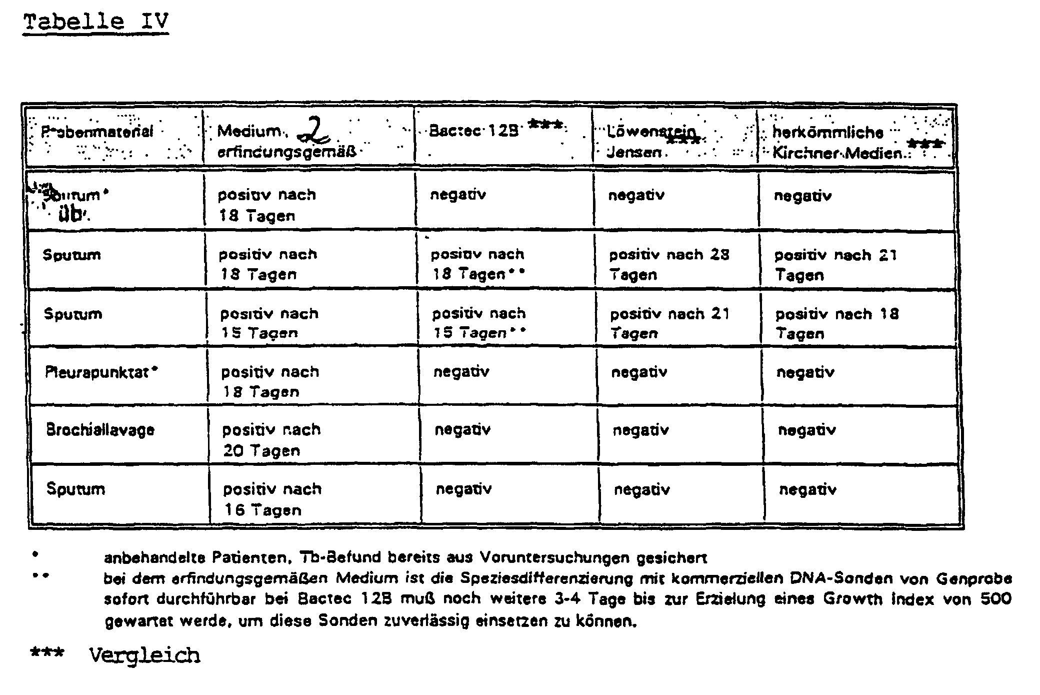

Tabelle IV zeigt, daß durch Anbehandlung geschädigte und gestreßte Keime in dem erfindungsgemäßen Medium noch anzüchtbar sind, während dies in den Vergleichsmedien nicht mehr der Fall ist.

Weiter ist die sofortige Weiterverarbeitung mit molekularbiologischen DNA-Sonden mit dem erfindungsgmäßen Medium am selben Tag, an dem der positive Befund des Wachstums erhoben wird, möglich. Damit ist die Speziesdifferenzierung und der sofortige Befund an den Kliniken möglich, während in traditionellen Systemen noch weiter gewartet werden muß, um genügend Keime zur Untersuchung mit kommerziellen DNA-Sonden zu haben.

Wie aus diesen Beispielen klar hervorgeht, führt das erfindungsgemäße Verfahren in einfacher, kostengünstiger Weise zu zuverlässigen schnellen Ergebnissen bezüglich des Wachstums und dessen Nachweises verschiedenster Spezies.

## Patentansprüche

1. Verfahren zur Verbesserung des Wachstums und des Nachweises von Bakterien, Hefen, Pilzen oder Kokken, **dadurch gekennzeichnet, daß** man dem Kulturmedium sterilfiltrierten Hefeextrakt und p-Iodonitrotetrazoliumviolett zusetzt.

2. Verfahren gemäß Anspruch 1, **dadurch gekennzeichnet, dass** das Wachstum und der Nachweis von Mykobakterien verbessert wird.

3. Verfahren gemäß Anspruch 2, **dadurch gekennzeichnet, dass** das Wachstum und der Nachweis von Stämmen von Mykobakterien tuberculosis verbessert wird.

4. Verfahren gemäß Anspruch 1, **dadurch gekennzeichnet, daß** das Wachstum und der Nachweis von Luftkeimen verbessert wird.

5. Verfahren gemäß einem der Ansprüche 1 bis 4, **dadurch gekennzeichnet, daß** man dem Kulturmedium 0,5-10 g/l steril filtrierten Hefeextrakt und 1-30 mg/l p-lodonitrotetrazoliumviolett zusetzt.

6. Verfahren gemäß einem der Ansprüche 1 bis 5, **dadurch gekennzeichnet, daß** man als Kulturmedium 7H9 Bouillon, 7H9 mit OADC (Oleic Acid, Albumin, Dextrose, Catalase) und PANTA, (Polymyxin, Amphotericin B, Nalidixic Acid, Trimethoprim, Azlocillin), Kirchner Medium mit Pferdeextrakt, Löwenstein Jensen-Medium oder Tryptic Soy Agar verwendet

## Claims

1. Method for improving the growth and the determination of bacteria, yeasts, fungi or cocci,
**characterised in that**
the culture medium is supplemented with sterile filtered yeast extract and p-iodonitrotetrazoliumviolet.

2. The method according to claim 1,
**characterised in that**
the growth and determination of mycobacteria is improved.

3. The method according to claim 2,
**characterised in that**
that the growth and determination of strains of *Mycobacteria tuberculosis* is improved.

4. The method according to claim 1,
**characterised in that**
the growth and determination of aerogerms is improved.

5. The method according to one of claims 1 to 4
**characterised in that**
the culture medium is supplemented with 0.5 to 10 g/l sterile filtered yeast extract and 1 to 30 mg/l p-iodonitrotetrazoliumviolet.

6. The method according to one of claims 1 to 5
**characterised in that**
7H9 bouillon, 7H9 bouillon with OADC (oleic acid, albumin, dextrose, catalase) and PANTA (polymyxin, amphotericin B, nalidixic acid, trimethoprim, aziocillin), Kirchner medium with horse extract, Lowenstein-Jensen-medium or tryptic soy agar are used as culture medium.

## Revendications

1. Procédé pour améliorer la croissance et la détection de bactéries, levures, champignons ou coccus, **caractérisé en ce que** l'on ajoute au milieu de culture, un extrait de levure filtré de manière stérile et du violet de p-iodonitrotétrazolium.

2. Procédé selon la revendication 1, **caractérisé en ce que** l'on améliore la croissance et la détection de mycobactéries.

3. Procédé selon la revendication 2, **caractérisé en ce que** l'on améliore la croissance et la détection de souches de *Mycobacterium tuberculosis.*

4. Procédé selon la revendication 1, **caractérisé en ce que** l'on améliore la croissance et la détection de germes d'air.

5. Procédé selon l'une quelconque des revendications 1 à 4, **caractérisé en ce que** l'on ajoute au milieu de culture, 0,5-10 g/litre d'extrait de levure filtré de manière stérile et 1-30 mg/litre de violet de p-iodonitrotétrazolium.

6. Procédé selon l'une quelconque des revendications 1 à 5, **caractérisé en ce que** l'on utilise comme milieu de culture, le bouillon 7H9, 7H9 avec OADC (acide oléique, albumine, dextrose, catalase) et PANTA (polymyxine, amphotéricine B, acide nalidixique, triméthoprim, azlocilline), le milieu de Kircher avec extrait de cheval, le milieu de Löwenstein Jensen ou la gélose de soya tryptique.
